# EUROPEAN PATENT APPLICATION

(11) **EP 4 616 806 A1**
(43) Date of publication of application: **17.09.2025**
(21) Application number: 25162452.4
(22) Date of filing: 07.03.2025
(51) Int. Cl.: A61B 6/00, A61B 8/00, A61B 6/42, A61B 6/50

(54) **MEDICAL IMAGE ACQUISITION APPARATUS AND IMAGE GENERATION SYSTEM**

(30) Priority: 11.03.2024 JP 2024037654
(71) Applicant: FUJI-FILM Corporation, Minato-ku Tokyo 106-8620 (JP)
(72) Inventor: OSAWA, Atsushi, Kaisei-machi (JP)
(74) Representative: Dehns Germany Partnerschaft mbB

(57) **Abstract**

There are provided a medical image acquisition apparatus and an image generation system capable of, in a case in which a radiation image and an ultrasound image of a breast in a state of being compressed by a compression member are captured, avoiding an influence of a device that performs one imaging on a device that performs the other imaging.

A medical image acquisition apparatus (10) captures a radiation image of a breast in a state of being compressed by a compression member (40) using a radiation image acquisition device and captures an ultrasound image using an ultrasound image acquisition device that transmits and receives an ultrasonic wave, in which an element group of the ultrasound image acquisition device and an element group of the radiation image acquisition device are arranged so as not to overlap at least partially with each other in an incidence direction of radiation (R).

## Description

### BACKGROUND OF THE INVENTION

### 1. Field of the Invention

The present disclosure relates to a medical image acquisition apparatus and an image generation system.

### 2. Description of the Related Art

Currently, as main examination methods for breast cancer, there are an examination using radiographic mammography and an ultrasound examination.

Due to differences in imaging principle, generally, a calcified lesion is easily visualized in the examination using the radiographic mammography, and a boundary of a soft tissue such as a tumor is easily visualized in the ultrasound examination. Therefore, attempts have been made to use a combination of the examination using the radiographic mammography and the ultrasound examination, but sensitivity is improved while specificity is decreased as compared with the single examination using the radiographic mammography. This is considered to be because both modalities introduce false positives, and a rate of cases requiring a detailed examination is increased.

In the combined examination of the examination using the radiographic mammography and the ultrasound examination, since a diagnosis is made by comparing a radiation image captured in a state where a breast is compressed with an ultrasound image acquired in a supine posture, a posture of a patient in a case of acquiring the image is different, and a shape of the breast is also different, so that it is difficult to specify or compare a position suspected to have a lesion, and the specificity is considered to be further decreased.

Therefore, it is considered to improve the specificity by performing an ultrasound examination without changing the posture of the patient in a state where the breast is compressed in the examination using the radiographic mammography and acquiring an ultrasound image via a compression member such as a compression plate. In this case, typically, radiography is performed first, followed by scanning of a suspicious part with an ultrasound examination.

However, in imaging using ultrasonic waves via the compression member, scanning is performed using ultrasonic waves via the compression member in which attenuation or refraction occurs with respect to the ultrasonic waves, so that the sensitivity and the definition are decreased due to the compression member, and a time required for acquiring an image of the entire breast is increased, making the work highly dependent on the individual.

As a technique that can be applied to solve these problems, JP2009-279111A discloses a medical imaging apparatus that aims to shorten a time required for obtaining an ultrasound image by using a two-dimensional ultrasound transducer array and obtaining a good radiation image even though radiography is performed with the array fixed.

The medical imaging apparatus includes a radiation generation unit that generates radiation, a radiation detection unit that detects the radiation, an ultrasound transducer array that includes a plurality of ultrasound transducers disposed in a two-dimensional manner and that is disposed between the radiation generation unit and the radiation detection unit, a radiation image data generation unit that generates radiation image data based on a detection result of the radiation detection unit, and an image processing unit that performs image processing on the radiation image data generated based on a detection result of the radiation that is generated by the radiation generation unit and that is transmitted through a subject and the ultrasound transducer array, to remove an image of the ultrasound transducer array from a radiation image represented by the radiation image data.

In addition, JP2008-173291A discloses a medical imaging apparatus that images a mammary gland or a breast using radiation and ultrasonic waves, and that aims to image a process in which the breast is compressed by a compression plate with ultrasonic waves and to shorten an imaging time by performing ultrasonic imaging simultaneously with radiography.

The medical imaging apparatus includes a radiation generation unit that generates radiation, an imaging table in which a radiation detection unit that detects the radiation generated by the radiation generation unit and passing through a subject is disposed, an ultrasound transducer array that is disposed between the radiation generation unit and the imaging table, allows a part of the radiation that has passed through the subject to pass therethrough, transmits ultrasonic waves toward the subject according to a plurality of drive signals, receives the ultrasonic waves reflected by the subject, and outputs a plurality of detection signals, and an ultrasonic examination unit that supplies the plurality of drive signals to the ultrasound transducer array and generates an image signal based on the plurality of detection signals output from the ultrasound transducer array.

### SUMMARY OF THE INVENTION

In the techniques disclosed in JP2009-279111A and JP2008-173291A, the ultrasound image is acquired without using the compression member, so that the above-described decrease in sensitivity and definition, and the like due to the compression member can be avoided. However, in these techniques, a device for acquiring an ultrasound image (hereinafter, referred to as an "ultrasound image acquisition device") is located on a breast side with respect to a device for acquiring a radiation image (hereinafter, referred to as a "radiation image acquisition device"). Therefore, in these techniques, the ultrasound image acquisition device physically affects the radiation image acquired by the radiation image acquisition device, and as a result, the quality of the radiation image deteriorates.

In order to avoid the deterioration in the quality of the radiation image, it is also considered to dispose the radiation image acquisition device on the breast side with respect to the ultrasound image acquisition device. However, in this case, the radiation image acquisition device physically affects the ultrasound image acquired by the ultrasound image acquisition device, and as a result, the quality of the ultrasound image deteriorates.

The present disclosure has been made in view of the above circumstances, and an object of the present disclosure is to provide a medical image acquisition apparatus and an image generation system capable of, in a case in which a radiation image and an ultrasound image of a breast in a state of being compressed by a compression member are captured, avoiding an influence of a device that performs one imaging on a device that performs the other imaging.

In order to achieve the above object, a first aspect of the present disclosure provides a medical image acquisition apparatus that captures a radiation image of a breast in a state of being compressed by a compression member using a radiation image acquisition device and that captures an ultrasound image using an ultrasound image acquisition device that transmits and receives an ultrasonic wave, in which an element group of the ultrasound image acquisition device and an element group of the radiation image acquisition device are arranged so as not to overlap at least partially with each other in an incidence direction of radiation.

A second aspect of the present disclosure provides the medical image acquisition apparatus according to the first aspect, in which the element group of the ultrasound image acquisition device and the element group of the radiation image acquisition device are arranged on the same substrate.

A third aspect of the present disclosure provides the medical image acquisition apparatus according to the first or second aspect, in which an image acquisition region of the ultrasound image acquisition device is substantially the same region as an image acquisition region of the radiation image acquisition device.

A fourth aspect of the present disclosure provides the medical image acquisition apparatus according to the first or second aspect, in which the ultrasound image acquisition device is created by a process of creating a silicon semiconductor device.

A fifth aspect of the present disclosure provides the medical image acquisition apparatus according to the first or second aspect, in which the ultrasound image acquisition device is an electrostatic capacitance type device that transmits and receives an ultrasound signal.

A sixth aspect of the present disclosure provides the medical image acquisition apparatus according to the first or second aspect, in which, in the ultrasound image acquisition device, elements for transmitting and receiving ultrasonic waves are arranged at a pitch calculated from a wavelength of the ultrasonic waves and beam steering, the pitch being equal to or smaller than a pitch at which no virtual image is reflected in the ultrasound image obtained by imaging.

A seventh aspect of the present disclosure provides the medical image acquisition apparatus according to the sixth aspect, in which, in the ultrasound image acquisition device, the elements for transmitting and receiving the ultrasonic waves are arranged at a random arrangement density.

In order to achieve the above object, an eighth aspect of the present disclosure provides an image generation system comprising: the medical image acquisition apparatus according to the present disclosure; and a console that controls the medical image acquisition apparatus.

According to the present disclosure, in a case in which a radiation image and an ultrasound image of a breast in a state of being compressed by a compression member are captured, it is possible to avoid an influence of a device that performs one imaging on a device that performs the other imaging.

### BRIEF DESCRIPTION OF THE DRAWINGS

Fig. 1 is a diagram showing an example of a schematic configuration of an image generation system according to an embodiment.
Fig. 2 is a side view showing an example of an appearance of a medical image acquisition apparatus according to the embodiment.
Fig. 3 is a three-plane view showing an example of a schematic configuration of a compression member according to the embodiment.
Fig. 4 is a perspective view showing an example of a schematic configuration of a radiation detector in the related art using an indirect conversion method.
Fig. 5 is a diagram showing an example of a partial configuration of an image detector according to the embodiment.
Fig. 6 is a diagram showing another example of the partial configuration of the image detector according to the embodiment.
Fig. 7 is a diagram showing still another example of the partial configuration of the image detector according to the embodiment.
Fig. 8 is a diagram showing an example of an overall configuration of the image detector according to the embodiment.
Fig. 9 is a block diagram showing an example of a hardware configuration of a console according to the embodiment.
Fig. 10 is a block diagram showing an example of a functional configuration of the console according to the embodiment.
Fig. 11 is a flowchart showing an example of image display processing according to the embodiment.

### DESCRIPTION OF THE PREFERRED EMBODIMENTS

Hereinafter, an embodiment of the present disclosure will be described with reference to the drawings.

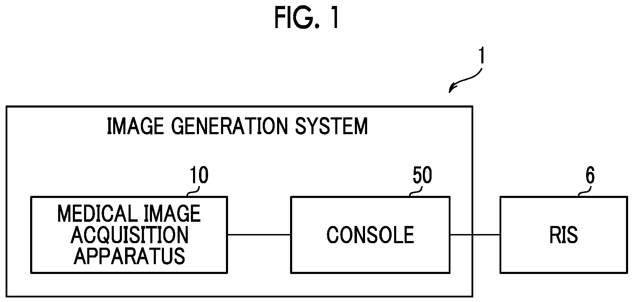

First, a configuration of an image generation system 1 to which a technique of the present disclosure is applied will be described with reference to Fig. 1. Fig. 1 is a diagram showing an example of a schematic configuration of the image generation system 1 according to the present embodiment.

As shown in Fig. 1, the image generation system 1 comprises a medical image acquisition apparatus 10 and a console 50. The medical image acquisition apparatus 10 and the console 50, and the console 50 and an external radiology information system (RIS) 6 are configured to be connected to each other via a wired or wireless network.

In the image generation system 1 according to the present embodiment, the console 50 acquires an imaging order or the like from the RIS 6, and controls the medical image acquisition apparatus 10 in accordance with the imaging order, an instruction from a user, and the like. The medical image acquisition apparatus 10 captures a radiation image by irradiating a breast put into a compressed state between an imaging table 16 and a compression member 40, both of which will be described below, with radiation R. In addition, the medical image acquisition apparatus 10 captures an ultrasound image of the breast put into the compressed state by the compression member 40.

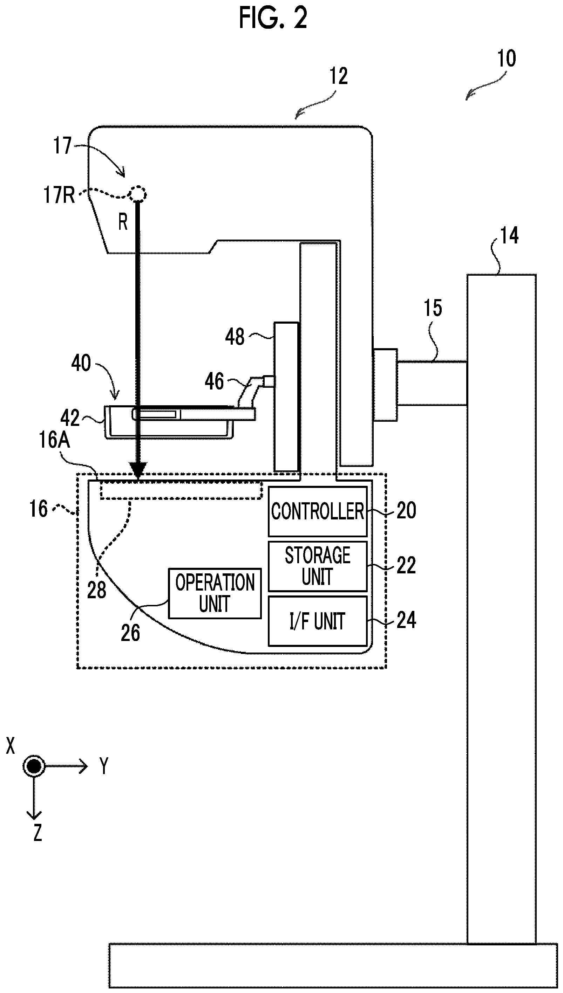

Next, a schematic configuration of the medical image acquisition apparatus 10 according to the present embodiment will be described with reference to Fig. 2. Fig. 2 is a side view showing an example of an appearance of the medical image acquisition apparatus 10 according to the present embodiment, and is a view of the medical image acquisition apparatus 10 as viewed from a right side of a subject. As shown in Fig. 2, the medical image acquisition apparatus 10 comprises a radiation source 17R, an image detector 28, an imaging table 16 disposed between the radiation source 17R and the image detector 28, and a compression member 40 that compresses the breast between the compression member 40 and the imaging table 16. In the medical image acquisition apparatus 10, a user, such as a doctor or a technician, positions the breast of the subject on an imaging surface 16A of the imaging table 16.

The medical image acquisition apparatus 10 comprises an arm part 12, a base 14, and a shaft part 15. The arm part 12 is held by the base 14 so as to be movable in an up-down direction (Z direction). The shaft part 15 connects the arm part 12 to the base 14. The arm part 12 is rotatable relative to the base 14, using the shaft part 15 as a rotation axis. In addition, the arm part 12 may be configured such that an upper part comprising a radiation emitting unit 17 and a lower part comprising the imaging table 16 are separately rotatable relative to the base 14, using the shaft part 15 as the rotation axis.

The arm part 12 comprises the radiation emitting unit 17 and the imaging table 16. The radiation emitting unit 17 comprises the radiation source 17R, and is configured to change an irradiation field of radiation (for example, X-rays) emitted from the radiation source 17R. The change of the irradiation field may be performed, for example, by the user operating an operation unit 26 or by a controller 20 in accordance with a type of the attached compression member 40.

The imaging table 16 comprises the controller 20, a storage unit 22, an interface (I/F) unit 24, the operation unit 26, and the image detector 28. The controller 20 controls an overall operation of the medical image acquisition apparatus 10 under the control of the console 50. The controller 20 comprises a central processing unit (CPU), a read only memory (ROM), a random access memory (RAM), and the like (not shown). The ROM stores in advance various programs various programs, including a program for controlling the generation of the radiation image and the ultrasound image, which are executed by the CPU. The RAM transitorily stores various data.

Data of the radiation image and the ultrasound image, various types of other information, and the like are stored in the storage unit 22. The storage unit 22 is implemented by, for example, a storage medium such as a hard disk drive (HDD), a solid state drive (SSD), and a flash memory.

The I/F unit 24 performs communication of various types of information with the console 50 through wired communication or wireless communication. Specifically, the I/F unit 24 receives information related to the control of the medical image acquisition apparatus 10 from the console 50. In addition, the I/F unit 24 transmits the data of the radiation image and the ultrasound image to the console 50.

The operation unit 26 is a part that is provided on the imaging table 16 or the like and that is operable by the user with a hand, a foot, or the like, and is, for example, a switch, a button, or a touch panel.

A compression unit 48 is connected to the arm part 12. A support part 46 that supports the compression member 40 is attachably and detachably attached to the compression unit 48. The support part 46 (compression member 40) is moved in the up-down direction (Z direction) by a driving unit (not shown) provided in the compression unit 48.

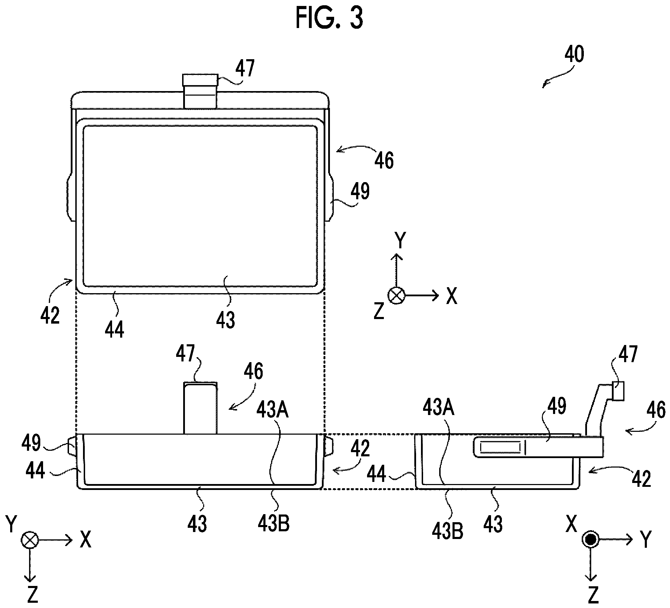

The compression member 40 is disposed between the radiation source 17R and the imaging table 16 and sandwiches the breast between the compression member 40 and the imaging table 16 to put the breast into a compressed state. Fig. 3 shows a three-plane diagram of an example of the compression member 40. The three-plane diagram of Fig. 3 includes a top view of the compression member 40 as viewed from above (radiation emitting unit 17 side), a side view thereof as viewed from a subject side, and a side view thereof as viewed from a right side of the subject. As shown in Fig. 3, the compression member 40 includes a compression part 42 and the support part 46.

The support part 46 includes an attachment portion 47 and an arm 49. The attachment portion 47 attaches the compression member 40 to the medical image acquisition apparatus 10, specifically, the driving unit of the compression unit 48. The arm 49 supports the compression part 42.

The compression part 42 includes a bottom portion 43 formed to be substantially flat and surrounded by a wall portion 44 having a substantially uniform height and has a recessed cross-sectional shape. The compression part 42 is preferably formed of an optically transparent or translucent material in order to perform positioning and confirmation of a compressed state in compression of the breast. In addition, the compression part 42 is preferably formed of a material having excellent transmittance of the radiation R. In addition, the compression part 42 is preferably formed of, for example, a material excellent in strength such as drop strength and compression strength.

As such a material, for example, a resin such as polymethylpentene (PMP), polycarbonate (PC), acryl, polypropylene (PP), and polyethylene terephthalate (PET) can be used.

In addition, a plurality of different types of compression members 40 may be attached to the medical image acquisition apparatus 10 in an interchangeable manner. Specifically, according to a physique of the subject (for example, a size of the breast), a tissue composition of the breast (for example, a fat mass and a mammary gland mass), a type of imaging (for example, magnified imaging and spot imaging), and the like, the compression members 40 having different materials, sizes, and shapes may be attached. For example, a compression member according to the size of the breast, a compression member for axillary imaging, a compression member for magnified imaging, a compression member for so-called spot imaging that captures a radiation image of only a region where a lesion exists, and the like may be used. That is, the compression member 40 is not limited to the compression member that compresses the entire breast, and may have a smaller size than the breast to compress a part of the breast.

On the other hand, the medical image acquisition apparatus 10 according to the present embodiment is configured to capture a radiation image of the breast in a state of being compressed by the compression member 40 by using a radiation image acquisition device and to capture an ultrasound image by using an ultrasound image acquisition device that transmits and receives an ultrasonic wave. In the medical image acquisition apparatus 10 according to the present embodiment, an element group of the ultrasound image acquisition device and an element group of the radiation image acquisition device are arranged so as not to overlap at least partially with each other in an incidence direction of the radiation R.

In particular, in the medical image acquisition apparatus 10 according to the present embodiment, the element group of the ultrasound image acquisition device and the element group of the radiation image acquisition device are arranged on the same substrate (in the present embodiment, a substrate of the image detector 28). The term "same substrate" here is not limited to a state where the substrate is formed in a completely uniform state, and need only be a state where the substrate can be treated as one substrate as a whole, and includes, for example, a substrate having a step or a substrate obtained by bonding a plurality of substrates.

The image detector 28 according to the present embodiment is disposed inside the imaging table 16, detects the radiation R transmitted through the breast and the imaging table 16, generates a radiation image based on the detected radiation R, and outputs image data representing the generated radiation image. In addition, the image detector 28 according to the present embodiment irradiates the breast with ultrasonic waves, receives reflected waves from the breast, generates an ultrasound image from the reflected waves, and outputs image data representing the generated ultrasound image.

Therefore, an imaging surface of the image detector 28 according to the present embodiment is provided with an element group of a radiation image acquisition device that detects the radiation R transmitted through the breast and the imaging table 16, and an element group of an ultrasound image acquisition device (in the present embodiment, an ultrasound transducer) that irradiates the breast with the ultrasonic waves and that receives the reflected waves from the breast.

A type of the radiation image acquisition device in the image detector 28 is not particularly limited, and may be, for example, an indirect conversion type device that converts the radiation R into light and that converts the converted light into an electric charge, or a direct conversion type device that directly converts the radiation R into an electric charge. In the following, a case in which an indirect conversion type device is applied as the radiation image acquisition device will be described.

In this configuration, it is required that the ultrasound image acquisition device does not suffer cumulative damage due to radiation. Therefore, as an ultrasound transducer, which is an element constituting the ultrasound image acquisition device, a capacitive micro-machined ultrasound transducer (cMUT), which is an ultrasound element that transmits and receives an ultrasound signal in an electrostatic capacitance type using an electrostatic material rather than a piezoelectric material and that is created in a process of creating a silicon semiconductor device, is suitable. Therefore, in the image detector 28 according to the present embodiment, the cMUT is applied as the ultrasound transducer.

Note that the ultrasound transducer constituting the ultrasound image acquisition device is not limited to the cMUT. For example, a bulk lead zirconate titanate (PZT) type using a piezoelectric material, a piezoelectric micro-machined ultrasound transducer (pMUT) to which piezoelectric thin film displacement is applied, or the like may be used as the ultrasound transducer constituting the ultrasound image acquisition device.

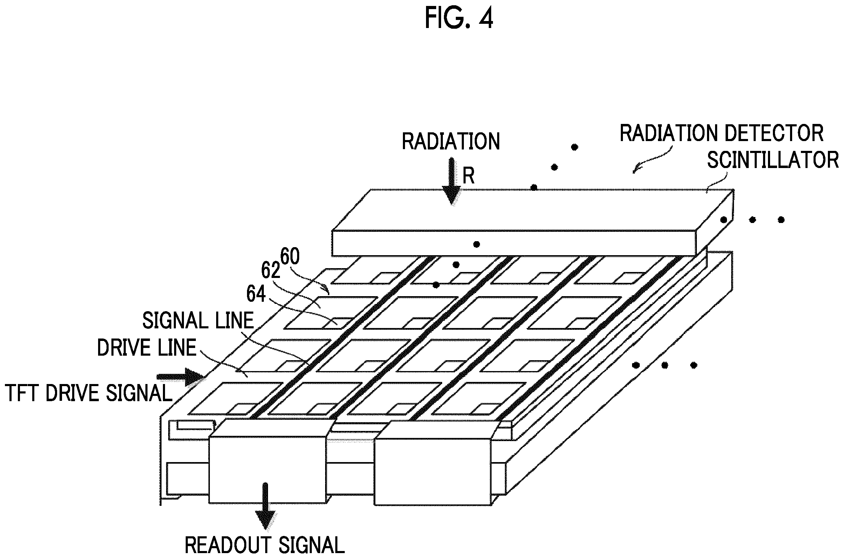

Fig. 4 is a perspective view showing an example of a schematic configuration of a radiation detector in the related art using an indirect conversion method. As shown in Fig. 4, the radiation detector in the related art using the indirect conversion method includes elements 60 each having a photodiode (PD) 62 and a thin film transistor (TFT) switch 64, and has an array structure of the elements 60. In the image detector 28 according to the present embodiment, a part of an imaging region of the radiation detector in the related art is changed to a region of the ultrasound transducer that is the cMUT.

An arrangement interval of the cMUT is decided by a wavelength of the ultrasonic waves to be transmitted and received, which has a longer wavelength than radiation or the like. For example, in a case in which the cMUT transmits and receives ultrasonic waves of 7 MHz, a pitch of 200 to 300 µm is sufficient, which is 1/2 to 1/3 or less of an arrangement pitch of the element group of the radiation image acquisition device.

In particular, in the ultrasound image acquisition device according to the present embodiment, elements are arranged at a pitch calculated from a wavelength of the ultrasonic waves transmitted and received by the elements and beam steering, the pitch being equal to or smaller than a pitch at which no virtual image is reflected in the ultrasound image obtained by imaging. This pitch is a pitch that is equal to or lower than a level at which no grating lobe is generated by the ultrasonic wave in a range from half of the wavelength of the ultrasonic wave to about one wavelength.

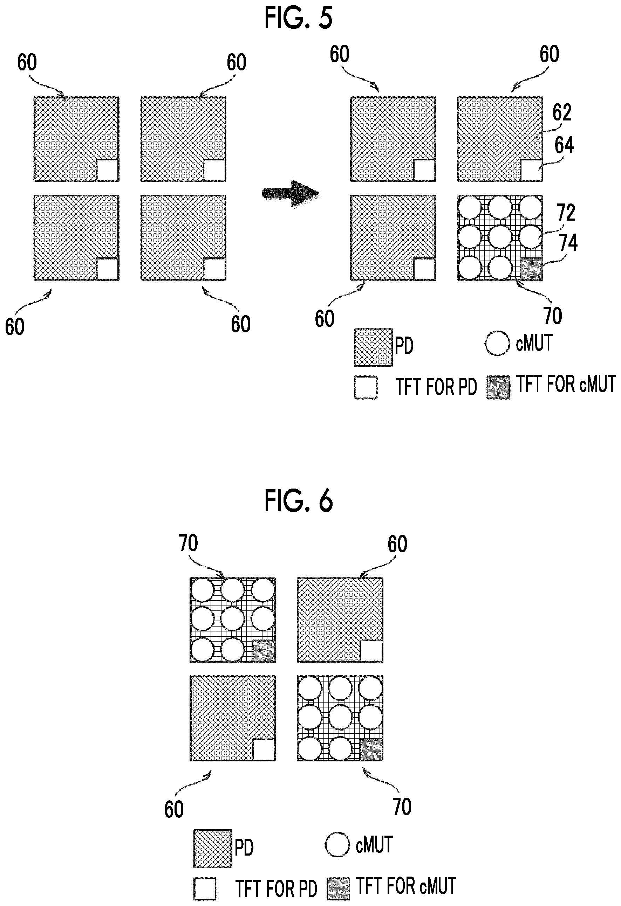

Fig. 5 is a diagram showing an example of a partial configuration of the image detector 28 according to the present embodiment. As shown in Fig. 5, the image detector 28 according to the present embodiment has a basic configuration in which three elements 60 of the radiation image acquisition device are arranged and one element 70 of the ultrasound image acquisition device is arranged with respect to pixels of a 2 × 2 region in an imaging region of the image detector 28. As shown in Fig. 5, the element 70 has a cMUT 72 and a TFT switch for cMUT 74.

In the image detector 28 according to the present embodiment, the basic configuration is randomly arranged relative to the array-like arrangement of the elements 60 in the radiation detector in the related art. As a result, it is possible to suppress image unevenness of the ultrasound image and the radiation image.

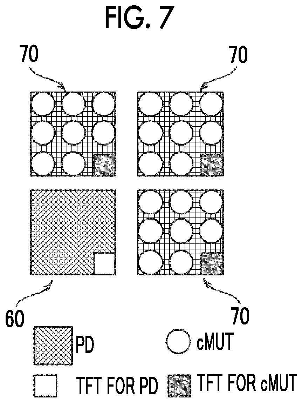

The basic configuration is not limited to the configuration shown in Fig. 5. As shown in Fig. 6, a configuration in which two elements 60 and two elements 70 are alternately arranged may be adopted, or as shown in Fig. 7, a configuration in which three elements 70 are arranged with respect to one element 60 may be adopted.

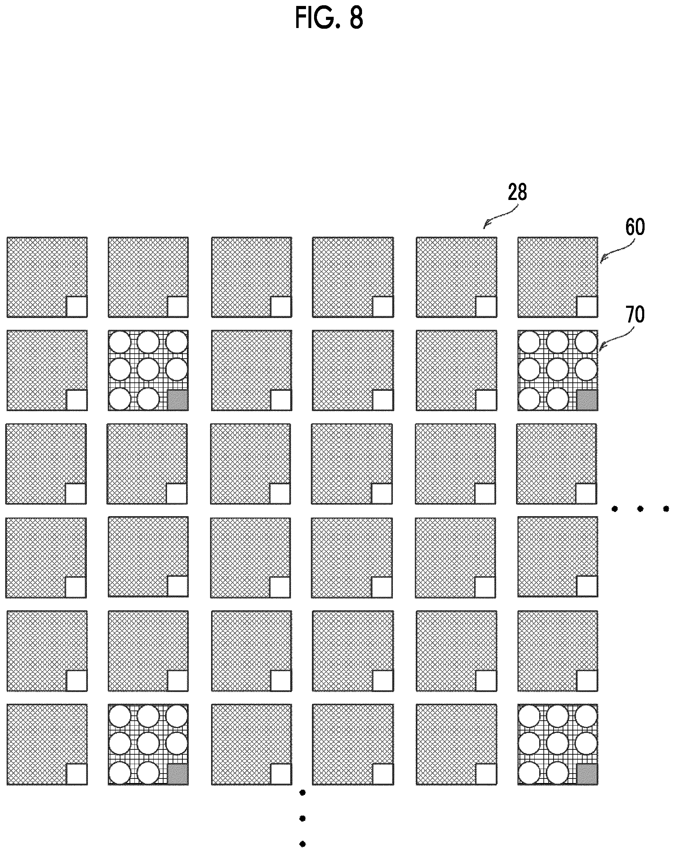

In addition, as shown in Fig. 8 as an example, an arrangement density of the elements 70 may be changed for each partial region with respect to the imaging region of the image detector 28. In the example shown in Fig. 8, an image acquisition region of the ultrasound image acquisition device is substantially the same region as an image acquisition region of the radiation image acquisition device.

A method of imaging the breast using the medical image acquisition apparatus 10 is not particularly limited. For example, cranio-caudal (CC) imaging, medio-lateral oblique (MLO) imaging, magnified imaging and spot imaging for imaging a part of the breast, and the like may be used. The CC imaging is a method of imaging the breast in a compressed state by sandwiching the breast between the imaging table 16 and the compression member 40 in the up-down direction (Z direction). The MLO imaging is a method of imaging the breast in a compressed state, including an axillary portion, by sandwiching the breast between the imaging table 16 and the compression member 40 in a tilted state in which a rotation angle of the arm part 12 with respect to the base 14 is 45 degrees or more and less than 90 degrees.

In addition, for example, the medical image acquisition apparatus 10 may perform tomosynthesis imaging. In the tomosynthesis imaging, the radiation source 17R irradiates the breast with the radiation R from each of a plurality of irradiation positions having different irradiation angles, and a plurality of radiation images of the breast are captured. That is, in the tomosynthesis imaging, the imaging is performed by changing a rotation angle of the radiation emitting unit 17 with respect to the base 14 while angles of the imaging table 16, the compression member 40, the breast, and the like are fixed.

In addition, in the medical image acquisition apparatus 10, the breast of the subject may be positioned not only in a state where the subject is standing up (standing state), but also in a state where the subject is sitting on a chair, a wheelchair, or the like (sitting state).

The console 50 sets an upper limit value of a compression force applied to the breast by the compression member 40 according to the type of the compression member 40 attached to the medical image acquisition apparatus 10. In addition, the console 50 controls the medical image acquisition apparatus 10 to acquire the radiation image and the ultrasound image in accordance with the imaging order acquired from the RIS 6 and the instruction from the user.

The medical image acquisition apparatus 10 according to the present embodiment can acquire both the radiation image and the ultrasound image by using one image detector 28 in a state where the breast is compressed by the compression member 40. As a result, registration between these images is facilitated, and a differential determination ability of the lesion can be improved by displaying both images in a superimposed manner.

Next, the console 50 according to the present embodiment will be described.

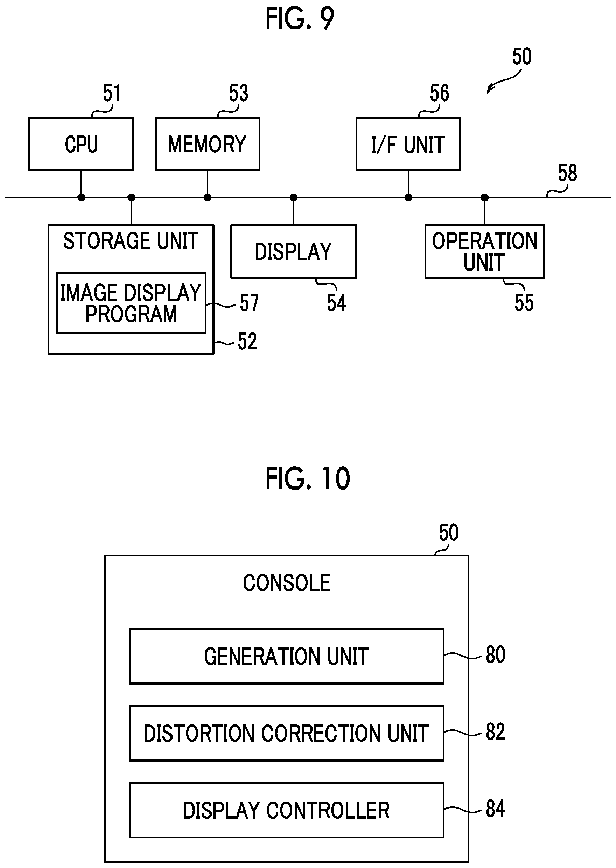

An example of a hardware configuration of the console 50 will be described with reference to Fig. 9. As shown in Fig. 9, the console 50 includes a CPU 51, a non-volatile storage unit 52, and a memory 53 as a transitory storage region. In addition, the console 50 includes a display 54 such as a liquid crystal display, an operation unit 55 such as a touch panel, a keyboard, and a mouse, and an I/F unit 56. The I/F unit 56 performs wired or wireless communication with the medical image acquisition apparatus 10, the RIS 6, other external devices, and the like. The CPU 51, the storage unit 52, the memory 53, the display 54, the operation unit 55, and the I/F unit 56 are connected to each other via a bus 58 such as a system bus and a control bus, so that various types of information can be exchanged.

The storage unit 52 is implemented by, for example, a storage medium such as an HDD, an SSD, and a flash memory. An image display program 57 is stored in the storage unit 52. The CPU 51 reads out the image display program 57 from the storage unit 52, loads the image display program 57 into the memory 53, and executes the loaded image display program 57. As the console 50, for example, a personal computer, a server computer, a smartphone, a tablet terminal, or a wearable terminal can be appropriately applied.

In addition, the storage unit 52 stores image data of the radiation image and the ultrasound image acquired by the medical image acquisition apparatus 10, various types of other information, and the like. The image data of the radiation image and the ultrasound image may be stored in association with at least one of an imaging order or imaging information. The imaging information may be, for example, at least one of subject information and an imaging item that are included in the imaging order, imaging person information indicating an imaging person (for example, a user such as a doctor or a technician) who performed the imaging, or date and time information indicating date and time when the imaging is performed.

An example of a functional configuration of the console 50 will be described with reference to Fig. 10. As shown in Fig. 10, the console 50 includes a generation unit 80, a distortion correction unit 82, and a display controller 84. The CPU 51 executes the image display program 57, so that the CPU 51 functions as the generation unit 80, the distortion correction unit 82, and the display controller 84.

The generation unit 80 according to the present embodiment generates the radiation image and the ultrasound image of the breast in a state where the compression member 40 is used. Then, the display controller 84 according to the present embodiment displays the ultrasound image and the radiation image generated by the generation unit 80 in a superimposed manner. As described above, in the present embodiment, the display controller 84 displays the ultrasound image and the radiation image generated by the generation unit 80 in a superimposed manner, but the present disclosure is not limited to this. For example, the display controller 84 may display the ultrasound image and the radiation image side by side.

On the other hand, in a case in which the display controller 84 performs the display, the distortion correction unit 82 according to the present embodiment performs distortion correction on at least one of the ultrasound image or the radiation image (in the present embodiment, both the ultrasound image and the radiation image).

That is, in a case in which the breast is compressed, the compression part 42 of the compression member 40 is distorted according to the compression force. Then, in the present embodiment, the distortion correction unit 82 performs distortion correction on both the ultrasound image and the radiation image.

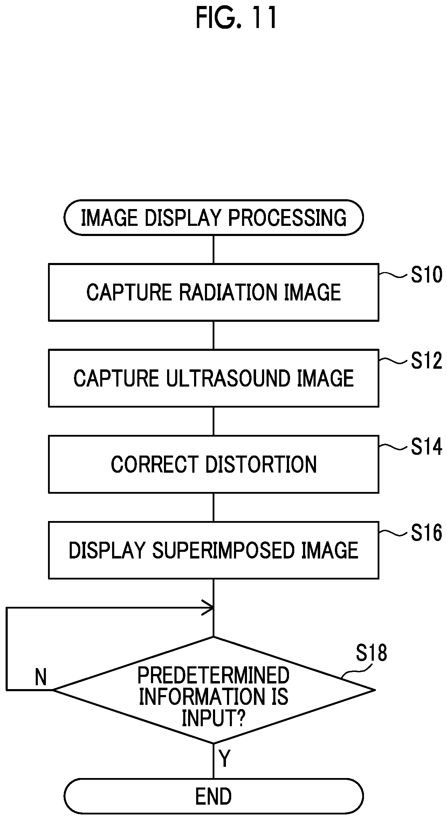

Next, an action of the console 50 according to the present embodiment will be described with reference to Fig. 11. In the console 50, the CPU 51 executes the image display program 57 to execute image display processing shown in Fig. 11. The image display processing is executed, for example, in a case in which the user gives an instruction to start the execution via the operation unit 55. Here, in order to avoid complications, a case is described in which the breast of the subject is positioned on the imaging surface 16A of the imaging table 16 by the user such as the doctor or the technician, and is put into a compressed state by the compression member 40.

In step S10, the CPU 51 controls the medical image acquisition apparatus 10 to capture a radiation image. In step S12, the CPU 51 controls the medical image acquisition apparatus 10 to capture an ultrasound image.

In step S14, the CPU 51 performs distortion correction on the radiation image and the ultrasound image obtained by the above processing. In step S16, the CPU 51 controls the display 54 to display the radiation image and the ultrasound image after the above processing in a superimposed manner, and, in step S18, the CPU 51 waits until predetermined information is input. Hereinafter, the image displayed in a superimposed manner will be referred to as a "superimposed image". By referring to the superimposed image, the user can more easily grasp the presence or absence of a lesion in the breast and the state of the lesion.

After referring to the superimposed image, the user uses the operation unit 55 to designate an end button displayed on a screen on which the superimposed image is displayed. In response to this designation, an affirmative determination is made in step S18, and the image display processing ends.

As described above, with the medical image acquisition apparatus according to the present embodiment, the element group of the ultrasound image acquisition device and the element group of the radiation image acquisition device are arranged so as not to overlap at least partially with each other in the incidence direction of the radiation. Accordingly, in a case in which the radiation image and the ultrasound image of the breast in a state of being compressed by the compression member are captured, it is possible to avoid the influence of a device that performs one imaging on a device that performs the other imaging.

In addition, with the medical image acquisition apparatus according to the present embodiment, the element group of the ultrasound image acquisition device and the element group of the radiation image acquisition device are arranged on the same substrate. Accordingly, it is possible to more reliably avoid the influence of a device that performs one imaging on a device that performs the other imaging as compared to a case in which the element group of the ultrasound image acquisition device is arranged on a different substrate from the element group of the radiation image acquisition device.

In addition with the medical image acquisition apparatus according to the present embodiment, the image acquisition region of the ultrasound image acquisition device is substantially the same region as the image acquisition region of the radiation image acquisition device. Accordingly, it is possible to more reliably acquire the ultrasound image for a region equivalent to that of the radiation image as compared with a case in which the ultrasound image is acquired by the ultrasound probe.

In addition, with the medical image acquisition apparatus according to the present embodiment, the ultrasound image acquisition device is created by the process of creating the silicon semiconductor device. Accordingly, it is possible to suppress the cumulative damage of the ultrasound image acquisition device due to the irradiation of the radiation as compared with a case in which a process of dividing piezoelectric ceramics or piezoelectric single crystals to create an ultrasound element is applied.

In addition, with the medical image acquisition apparatus according to the present embodiment, the ultrasound image acquisition device is an electrostatic capacitance type device that transmits and receives the ultrasound signal. Accordingly, it is possible to suppress the cumulative damage of the ultrasound image acquisition device due to the irradiation of the radiation as compared with a case in which a device that transmits and receives the ultrasound signal using the ultrasound element created from the piezoelectric ceramics or the piezoelectric single crystals is used.

In addition, with the medical image acquisition apparatus according to the present embodiment, in the ultrasound image acquisition device, the elements for transmitting and receiving the ultrasonic waves are arranged at the pitch calculated from the wavelength of the ultrasonic waves and the beam steering, the pitch being equal to or smaller than the pitch at which no virtual image is reflected in the ultrasound image obtained by imaging. Accordingly, it is possible to prevent the virtual image from being reflected in the ultrasound image.

In addition, with the medical image acquisition apparatus according to the present embodiment, the elements for transmitting and receiving the ultrasonic waves in the ultrasound image acquisition device are arranged at a random arrangement density. Accordingly, it is possible to further suppress the image unevenness as compared with a case in which the elements that transmit and receive the ultrasonic waves are arranged at a regular arrangement density.

Further, with the image generation system according to the present embodiment, the medical image acquisition apparatus as described above is provided, and the radiation image and the ultrasound image are generated by using the medical image acquisition apparatus. Accordingly, as with the medical image acquisition apparatus, in a case in which the radiation image and the ultrasound image of the breast in a state of being compressed by the compression member are captured, it is possible to avoid the influence of a device that performs one imaging on a device that performs the other imaging.

In the above-described embodiment, a case has been described in which the element group of the radiation image acquisition device and the element group of the ultrasound image acquisition device are arranged on the same substrate of the image detector 28, but the present disclosure is not limited to this. For example, a form may be adopted in which the element group of the radiation image acquisition device and the element group of the ultrasound image acquisition device are arranged on different substrates, and these substrates are superimposed on each other such that the element group of the ultrasound image acquisition device and the element group of the radiation image acquisition device do not overlap at least partially with each other in the incidence direction of the radiation.

In the above-described embodiment, for example, as a hardware structure of a processing unit that executes various types of processing such as the generation unit 80, the distortion correction unit 82, and the display controller 84, various processors shown below can be used. The various processors include, as described above, in addition to a CPU, which is a general-purpose processor that functions as various processing units by executing software (program), a programmable logic device (PLD) that is a processor of which a circuit configuration may be changed after manufacture, such as a field programmable gate array (FPGA), and a dedicated electrical circuit which is a processor having a circuit configuration specially designed to execute specific processing, such as an application specific integrated circuit (ASIC).

One processing unit may be configured of one of the various processors, or may be configured of a combination of the same or different kinds of two or more processors (for example, a combination of a plurality of FPGAs or a combination of the CPU and the FPGA). In addition, a plurality of processing units may be configured of one processor.

As an example in which a plurality of processing units are configured of one processor, first, as typified by a computer such as a client or a server, there is an aspect in which one processor is configured of a combination of one or more CPUs and software, and this processor functions as a plurality of processing units. Second, as typified by a system on chip (SoC) or the like, there is an aspect in which a processor that implements functions of the entire system including the plurality of processing units via one integrated circuit (IC) chip is used. As described above, various processing units are configured by using one or more of the various processors as a hardware structure.

Further, as the hardware structure of the various processors, more specifically, an electric circuit (circuitry) in which circuit elements such as semiconductor elements are combined may be used.

In addition, in the above-described embodiment, the aspect is described in which the image display program 57 is stored (installed) in the storage unit 52 of the console 50 in advance, but the present disclosure is not limited to this. The image display program 57 may be provided in a form of a recording on a recording medium such as a compact disc read only memory (CD-ROM), a digital versatile disc read only memory (DVD-ROM), and a universal serial bus (USB) memory. In addition, the image display program 57 may be provided in a form of a download from an external device via a network.

From the above description, the invention described in following Appendices can be understood.

## Claims

1. A medical image acquisition apparatus (10) that captures a radiation image of a breast in a state of being compressed by a compression member (40) using a radiation image acquisition device and that captures an ultrasound image using an ultrasound image acquisition device that transmits and receives an ultrasonic wave,
wherein an element group of the ultrasound image acquisition device and an element group of the radiation image acquisition device are arranged so as not to overlap at least partially with each other in an incidence direction of radiation (R).

2. The medical image acquisition apparatus (10) according to claim 1,
wherein the element group of the ultrasound image acquisition device and the element group of the radiation image acquisition device are arranged on the same substrate.

3. The medical image acquisition apparatus (10) according to claim 1 or 2,
wherein an image acquisition region of the ultrasound image acquisition device is substantially the same region as an image acquisition region of the radiation image acquisition device.

4. The medical image acquisition apparatus (10) according to claim 1 or 2,
wherein the ultrasound image acquisition device is created by a process of creating a silicon semiconductor device.

5. The medical image acquisition apparatus (10) according to claim 1 or 2,
wherein the ultrasound image acquisition device is an electrostatic capacitance type device that transmits and receives an ultrasound signal.

6. The medical image acquisition apparatus (10) according to claim 1 or 2,
wherein, in the ultrasound image acquisition device, elements (60) for transmitting and receiving ultrasonic waves are arranged at a pitch calculated from a wavelength of the ultrasonic waves and beam steering, the pitch being equal to or smaller than a pitch at which no virtual image is reflected in the ultrasound image obtained by imaging.

7. The medical image acquisition apparatus (10) according to claim 6,
wherein, in the ultrasound image acquisition device, the elements (60) for transmitting and receiving the ultrasonic waves are arranged at a random arrangement density.

8. An image generation system (1) comprising:
the medical image acquisition apparatus (10) according to claim 1 or 2; and
a console (50) that controls the medical image acquisition apparatus (10).
